(19) Europäisches Patentamt
European Patent Office
Office européen des brevets

(11) Veröffentlichungsnummer: **0 065 707**
**B1**

(12) # EUROPÄISCHE PATENTSCHRIFT

(45) Veröffentlichungstag der Patentschrift: **17.04.85**

(51) Int. Cl.⁴: **C 07 C 45/45,** C 07 C 49/403 // C07C69/716

(21) Anmeldenummer: **82104156.3**

(22) Anmeldetag: **12.05.82**

(54) **Verfahren zur Herstellung von Dimedon.**

(30) Priorität: **27.05.81 CH 3473/81**

(43) Veröffentlichungstag der Anmeldung: **01.12.82 Patentblatt 82/48**

(45) Bekanntmachung des Hinweises auf die Patenterteilung: **17.04.85 Patentblatt 85/16**

(84) Benannte Vertragsstaaten: **AT BE CH DE FR GB IT LI LU NL SE**

(56) Entgegenhaltungen:
**FR - A - 2 200 230**
**FR - A - 2 319 609**

(73) Patentinhaber: **LONZA AG, Gampel/Wallis (CH)**

(72) Erfinder: **Lehky, Pavel, Dr., Dammweg 13, Naters (Kanton Wallis) (CH)**

(74) Vertreter: **Weinhold, Peter, Dr. et al, Patentanwälte Dr. V. Schmied-Kowarzik Dipl.-Ing. G. Dannenberg Dr. P. Weinhold Dr. D. Gudel Dipl.-Ing. S. Schubert Dr. P. Barz Siegfriedstrasse 8, D-8000 München 40 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 065 707 B1

BUNDESDRUCKEREI BERLIN

**Beschreibung**

Die Erfindung betrifft ein Verfahren zur Herstellung von Dimedon aus Isophoron.

Dimedon findet als Zwischenprodukt in der chemischen Industrie, wie bei der Herstellung von Pharmazeutika (US-PS 3 775 435, US-PS 3 823 164), Herbiziden resp. Pestiziden (DE-OS 2 201 668, US-PS 3 976 785) und Polymerisaten (JP-PS 7 747 949), in der Photochemie (US-PS 2 944 899, JP-PS 7 833 143) und in der Analytik (DE-OS 2 512 586) vielseitige Anwendungsmöglichkeiten.

Für die Herstellung von Dimedon sind aus der Literatur verschiedene Methoden bekannt. Aus der DE-PS 2 245 270 sowie aus der DE-OS 2 533 920 gehen z. B. Verfahren hervor, welche 1,3-Cyclohexandione allgemein durch Umsetzung von 4-Oxocarbonsäureestern mit Alkalialkoholaten geben. In Org. Synth., Coll. II, 200 (1943), wird ein Verfahren erwähnt, welches von Malonester und Mesityloxid ausgeht. T. Henschall et al., J. Amer. Chem. Soc. 77, 6656 (1955), erhalten Dimedon durch Zyklisation von 3,3-Dimethyl-5-oxo-hexansäure mit Schwefelsäure. G. B. Payne, J. Org. Chem. 24, 719 (1959), stellt Dimedon aus Isopheron mit Hilfe eines mehrstufigen, komplizierten Verfahrens her:

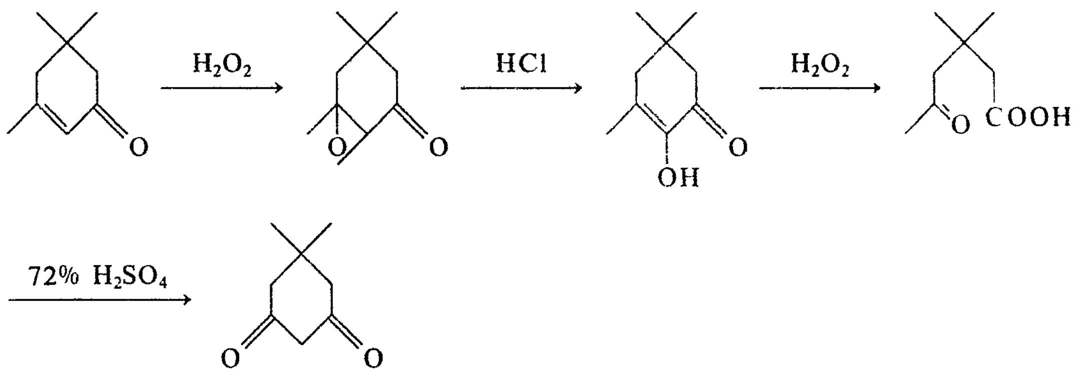


Alle diese Verfahren haben verschiedene Nachteile, wie tiefe Ausbeuten, schlechte Zugänglichkeit zur 3,3-Dimethyl-5-oxohexansäure, viel anorganischer Abfall aber besonders im letzteren Fall, viele einzelne Reaktionsstufen.

Ziel der vorliegenden Erfindung ist es, Dimedon, ausgehend von einem billigen Edukt, auf einfache Weise in hoher Reinheit zu produzieren, ohne daß noch zusätzliche, aufwendige Reinigungsverfahren angewendet werden müssen.

Erfindungsgemäß wird dies dadurch erreicht, daß man von Isophoron ausgeht, dieses in einem Lösungsmittel löst, mit Ozon in ein Ozonanlagerungsprodukt überführt, mit Hilfe eines spätestens nach der Bildung des Ozonanlagerungsproduktes zugesetzten Alkohols und einer vorzugsweise starken Säure in einen als Zwischenprodukt benötigten 3,3-Dimethyl-5-oxo-hexansäureester, z. B. dem Methyl- oder Äthylester, umwandelt und letzteren, nach dessen Isolierung, in einer zweiten Stufe durch Zugabe eines Alkalialkoholates in das gesuchte Dimedon umsetzt.

Die erfindungsgemäße Umsetzung kann in der ersten Stufe diskontinuierlich oder kontinuierlich durchgeführt werden.

Nach einer speziellen Ausführungsform des erfindungsgemäßen Verfahrens wird der erste Teil der ersten Stufe in Gegenwart von Lösungsmitteln durchgeführt. Diese Lösungsmittel können dem Esterrest des 3,3-Dimethyl-5-oxo-hexansäureesters entsprechende Alkohole sein, oder aber Lösungsmittel in der Art von Kohlenwasserstoffen, halogenierten Kohlenwasserstoffen, Estern wie Essigester und andere. Kommen letztere zum Einsatz, so ist es nötig, nach der Bildung des Ozonanlagerungsproduktes die genannten Alkohole zuzusetzen, um eine Alkoholyse des Ozonanlagerungsproduktes zum Ester zu erreichen.

Isophoron kann auch ohne Lösungsmittel mit Ozon umgesetzt werden. Bei dieser Arbeitsweise wird man aus Sicherheitsgründen darauf achten, daß nur 20 bis 30% des eingesetzten Isophorons in ein Ozonanlagerungsprodukt überführt werden. Die Alkoholyse des Ozonanlagerungsproduktes erfolgt auch in diesem Fall durch Zugabe eines Alkohols.

Vorzugsweise werden als Lösungsmittel dem Ersterrecht des 3,3-Dimethyl-5-oxo-hexansäureesters entsprechende Alkohole eingesetzt. Als starke Säuren kommen vorteilhaft Schwefel- und Salzsäure (insbesondere entsprechende konzentrierte Säure) und saure Ionenaustauscher in Betracht.

Die Ozonolyse wird zweckmäßig bei Temperaturen von $-80$ bis $+50°C$ durchgeführt, wobei der bevorzugte Bereich zwischen $-10$ bis $+30°C$ liegt.

Die Dauer der Ozonolyse hängt zur Hauptsache von der Leistungsfähigkeit des Ozongenerators ab und kann zwischen einigen Minuten und Stunden liegen. Mittlere Reaktionszeiten liegen etwa bei 0,5 bis 5 Stunden.

Der Konzentration des Isophorons im Lösungsmittel fällt nur sekundäre Bedeutung zu. Sie hat nur einen kleinen Einfluß auf die Ausbeuten und liegt zweckmäßig zwischen 5 und 35%.

Die Dauer der Nachbehandlung, der Veresterung, des Ozonanlagerungsproduktes ist von der Temperatur abhängig und liegt zwischen 0,5 und 25 Stunden. Zweckmäßig wird bei Temperaturen von 20 bis 150° C gearbeitet, wobei dann die mittlere Reaktionszeit 3 Stunden beträgt.

Die Isolierung des 3,3-Dimethyl-5-oxo-hexansäureesters kann nach einer bekannten Methode, wie Destillation, Extraktion etc., durchgeführt werden.

In der zweiten Stufe, d. h. bei der Umwandlung des isolierten Esters in Dimedon, kann wie folgt vorgegangen werden: In ein mit Rührer, Rückflußkühler und Zutropfvorrichtung ausgerüstetes geeignetes Gefäß wird eine Natriumalkoholat-Lösung in Alkohol vorgelegt. Diese Lösung enthält vorzugsweise 1,2 bis 1,5 Äquivalente Alkoholat, bezogen auf den eingesetzten 3,3-Dimethyl-5-oxo-hexansäureester, doch wird auch mit tieferen oder höheren Mengen an Alkoholat Dimedon gebildet. Zur Alkoholatlösung wird der Ester langsam zugetropft, das Ganze während 1 bis 2 Stunden auf Rückflußtemperatur gehalten, dann mit Wasser versetzt und mit einer starken Säure angesäuert. Vorteilhaft wird als starke Säure Schwefelsäure oder Salzsäure eingesetzt und die Ansäuerung so weit getrieben, bis ein pH von ca. 2 erreicht wird. Nach dem Ansäuern wird der Alkohol abdestilliert und die verbleibende wäßrige Lösung abgekühlt. Dabei kristallisiert das Dimedon als farblose Substanz aus.

## Beispiel 1

### 1. Stufe

Eine Lösung von 200 g Isophoron in 1500 g Methanol wurde während 20 Stunden oben in eine Glockenbodenkolonne kontinuierlich eingeleitet. Von unten wurde Sauerstoff, der ca. 4% Ozon enthielt, zugeführt. Flüssigphase und Gasphase bewegten sich somit im Gegenstrom.

Die Kolonne wurde auf der Außenseite durch einen Kühlmantel auf 0 bis 20° C gekühlt. Die aus der Kolonne unten ablaufende Lösung wurde kontinuierlich mit 6,4 g konz. Schwefelsäure vermischt und zum Rückfluß aufgeheizt. Nachdem die Ozonolyse beendet war, wurde die Reaktionslösung noch 3 Stunden lang unter Rückfluß gekocht. Anschließend wurde das Ganze auf 20° C abgekühlt und mit in Methanol gelöstem Natriumhydroxid neutralisiert. Der Methylalkohol wurde unter normalem Druck abdestilliert. Der gebildete Ester wurde durch Vakuumdestillation isoliert (Sdp. 80 bis 82° C/14 mbar).

Es wurden 231,4 g einer farblosen bis leicht gelben Flüssigkeit erhalten, die laut Gaschromatographie 98,1% 3,3-Dimethyl-5-oxo-hexansäuremethylester enthielt. Dies entsprach 227,0 g Ester 100%ig und einer Ausbeute von 91,1%, auf das eingesetzte Isophoron berechnet.

### 2. Stufe

In einem Rundkolben, mit Rührer, Rückflußkühler, $CaCl_2$-Rohr und Tropftrichter ausgerüstet, wurden 4,8 g metallisches Natrium in 60 $cm^3$ Methanol gelöst und das Ganze bis zum Rückfluß erwärmt. Dann wurden 30,0 g des 98,1%igen, aus der 1. Stufe erhaltenen 3,3-Dimethyl-5-oxo-hexansäuremethylesters langsam zugetropft und die Lösung während 2 Strunden weiter bei Rückflußtemperatur behandelt.

Nach Abkühlen auf 25° C wurde mit 150 ml $H_2O$ versetzt und mit konz. Salzsäure auf einen pH-Wert von ca. 2 eingestellt. Über einen Claisen-Aufsatz wurde unter Normaldruck das Methanol abdestilliert und der Rückstand anschließend auf 10° C abgekühlt. Dabei fiel das Dimedon in Form farbloser Kristalle aus. Das Produkt wurde abfiltriert, auf der Nutsche mit wenig Wasser gewaschen und bei 40° C/20 mbar getrocknet.

Man erhielt 21,07 g Dimedon, Smp. 147 bis 148° C, das laut potentiometrischer Titration einen Gehalt von 98,2% aufwies, entsprechend 20,69 g 100%ig, entsprechend einer Ausbeute von 86,4%, berechnet auf den eingesetzten Ester, resp. von 78,8%, berechnet auf das Isophoron.

In der Mutterlauge ließen sich (mit Gaschromatographie) weitere 1,85 g Dimedon nachweisen. Die gesamthaft gebildete Menge Dimedon betrug somit 22,54 g, entsprechend 94,1% Ausbeute, bezogen auf eingesetzten Ester, resp. 85,8%, bezogen auf eingesetztes Isophoron.

## Beispiel 2

Eine Lösung von 15,8 g Isophoron in 55,0 g Methanol wurde bei −2 bis 0° C 2 Stunden lang ozonolysiert. Nach der Zugabe von 0,91 g konz. Schwefelsäure wurde die Reaktionslösung 16 Stunden unter Rückfluß gekocht. Der pH der Lösung wurde mit einer N 2 methanolischen Lösung von Natriumhydroxid auf pH 7 eingestellt und das Methanol abdestilliert. Der gebildete Ester wurde durch Vakuumdestillation isoliert.

Es wurden 17,2 g einer leicht gelben Flüssigkeit (Sdp. 80 bis 82° C/14 mbar) erhalten, die laut Gaschromatographie 97,2% 3,3-Dimethyl-5-oxo-hexansäuremethylester enthielt. Dies entsprach 16,7 g Ester 100%ig und einer Ausbeute von 84,8%, auf eingesetztes Isophoron berechnet.

Beispiel 3

Eine Lösung von 15,8 g Isophoron in 70,0 g Äthylacetat wurde bei 0° C 2 Stunden lang ozonolysiert. Nach der Zugabe von 80,0 g abs. Äthanol und 0,91 g konz. Schwefelsäure wurde die Reaktionslösung über Nacht unter Rückfluß gekocht. Die abgekühlte Lösung wurde mit einer äthanolischen Lösung von Natriumhydroxid auf pH 7 eingestellt. Das Äthanol und das Äthylacetat wurden abdestilliert. Der erwünschte 3,3-Dimethyl-5-oxo-hexansäureäthylester wurde durch Vakuumdestillation isoliert. Man erhielt 18,6 g einer farblosen Flüssigkeit (Sdp. 104 bis 105° C/19 mbar), die laut Gaschromatographie 98,2% 3,3-Dimethyl-5-oxo-hexansäureäthylester enthielt. Dies entsprach 18,3 g Ester 100%ig und einer Ausbeute von 85,9%, auf das eingesetzte Isophoron berechnet.

Beispiel 4

In einer Apparatur, bestehend aus Rundkolben, Rückflußkühler mit Chlorcalciumrohr und Tropftrichter, wurden 3,8 g festes Natriummethylat in 20 ml trockenem Dimethylsulfoxid auf 80 bis 100° C erwärmt. Man ließ innerhalb von 10 Minuten 10,215 g (59,3 mmol) 3,3-Dimethyl-5-oxo-hexansäure-methylester, der wie in einem der vorstehenden Beispiele hergestellt wurde, zutropfen. Man digerierte die Lösung noch während 2 Stunden unter Beibehaltung der angegebenen Temperatur, kühlte auf Raumtemperatur und versetzte mit 80 ml Wasser.

Diese Lösung wurde mit konz. Salzsäure auf pH 2 eingestellt. Dabei fiel das Produkt als farbloser Kristallbrei an, welcher auf der Nutsche abfiltriert, mit wenig Wasser gewaschen und im Vakuumtrokkenschrank bei 40° C getrocknet wurde.

Man erhielt 6,622 g Dimedon in einer Reinheit von 98,6%, entsprechend 6,529 g 100%iges Dimedon, entsprechend einer Ausbeute von 78,5%, bezogen auf eingesetzten 3,3-Dimethyl-5-oxo-hexansäuremethylester.

Beispiel 5

In einem 100-ml-Rundkolben mit Rückflußkühler, Chlorcalciumrohr und Tropftrichter wurden 3,7 g festes Natriummethylat in 20 ml trockenem Acetonitril zum Rückfluß erwärmt. Man tropfte 9,873 g (0,057 mol) 3,3-Dimethyl-5-oxo-hexansäure-methylester, der wie in einem der vorstehenden Beispiele hergestellt wurde, innerhalb von 10 Minuten hinzu und beließ anschließend während 2 Stunden unter Rückfluß. Das erhaltene Reaktionsgemisch wurde am Rotationsverdampfer eingeengt und der Eindampfrückstand in 60 ml Wasser gelöst und mit konz. Salzsäure auf pH 2 eingestellt. Das ausgefallene Dimedon wurde abfiltriert, auf der Nutsche mit wenig Wasser gewaschen und im Vakuumtrockenschrank bei 40° C getrocknet.

Man erhielt 6,728 g Dimedon als farblose Kristalle in einer Reinheit von 98,8% (potentiometrische Titration), entsprechend 6,647 g Produkt 100%ig, entsprechend einer Ausbeute von 83,2%, bezogen auf eingesetzten 3,3-Dimethyl-5-oxo-hexansäure-methylester.

**Patentansprüche**

1. Verfahren zur Herstellung von Dimedon aus Isophoron, dadurch gekennzeichnet, daß man Isophoron, vorzugsweise in Gegenwart von Lösungsmitteln, mit Ozon in einer ersten Stufe in ein Ozonanlagerungsprodukt überführt, wobei für die Bildung des Zwischenproduktes 3,3-Dimethyl-5-oxo-hexansäureester ein dem Esterrest entsprechender Alkohol spätestens nach der Bildung des Ozonanlagerungsproduktes zugesetzt wird, daß das Ozonanlagerungsprodukt in Gegenwart von Säuren auf Temperaturen von 20 bis 150° C erwärmt wird, daß der sich dabei bildende 3,3-Dimethyl-5-oxo-hexansäureester isoliert wird und daß dieser Ester in einer weiteren Stufe durch Behandlung mit einem Alkalialkoholat in wasserfreiem Milieu in das Dimedon übergeführt wird.

2. Verfahren gemäß Patentanspruch 1, dadurch gekennzeichnet, daß als Lösungsmittel ein dem Esterrest entsprechender Alkohol verwendet wird und dieser schon vor der Bildung des Ozonanlagerungsproduktes dem Reaktionsgemisch zugesetzt wird.

3. Verfahren gemäß Patentansprüchen 1 bis 2, dadurch gekennzeichnet, daß man die erste Stufe kontinuierlich durchführt.

4. Verfahren gemäß Patentansprüchen 1 bis 3, dadurch gekennzeichnet, daß man die Ozonolyse bei Temperaturen von −80 bis +50° C durchführt.

5. Verfahren gemäß Patentansprüchen 1 bis 4, dadurch gekennzeichnet, daß die Konzentration des Isophorons im Lösungsmittel zwischen 2 und 100%, bevorzugt aber zwischen 3 und 35% liegt.

## Claims

1. Process for the preparation of dimedone from isophorone, characterized in that isophorone is, preferably n the presence of solvents, transformed with ozone in a first step into an ozone addition product, adding for the formation of the intermediate 3,3-dimethyl-5-oxo-hexanoic acid ester an alcohol corresponding to the ester radical at the latest after the formation of the ozone addition product, that said ozone addition product is heated to temperatures of 20 to 150° C in the presence of acids, that the thus forming 3,3-dimethyl-5-oxo-hexanoic acid ester is isolated and that said ester, in a further stept, is transformed into the dimedone by treatment with an alkali alcoholate in a water-free medium.

2. Process according to patent claim 1, characterized in that as solvent an alcohol corresponding to the ester radical is used and said alcohol is added to the reaction mixture already before the formation of the ozone addition product.

3. Process according to patent claims 1 and 2, characterized in that the first step is carried out continuously.

4. Process according to patent claims 1 to 3, characterized in that the ozonolysis is carried out at temperatures of −80 to +50° C.

5. Process according to patent claims 1 to 4, characterized in that the concentration of the isophorone in the solvent is between 2 and 100%, preferably between 3 to 35%.

## Revendications

1. Procédé pour la préparation de la dimédone à partir de l'isophorone, caractérisé en ce qu'on transforme l'isophorone, de préférence en présence de solvants, au moyen d'ozone dans une première étape en un produit de fixation de l'ozone, en ajoutant pour la formation du produit intermédiaire ester d'acide 3,3-diméthyl-5-oxo-hexanoïque un alcool correspondant au radical ester au plus tard après la formation du produit de fixation de l'ozone, en ce qu'on chauffe, à des températures de 20 à 150° C, le produit de fixation de l'ozone en présence d'acides, en ce qu'on isole l'ester d'acide 3,3-diméthyl-5-oxo-hexanoïque qui se forme alors et en ce qu'on transforme cet ester en dimédone dans une autre étape, par traitement avec un alcoolate alcalin en milieu anhydre.

2. Procédé selon la revendication 1, caractérisé en ce qu'on utilise comme solvant un alcool correspondant au radical ester et en ce qu'on ajoute cet alcool au mélange réactionnel déjà avant la formation du produit de fixation de l'ozone.

3. Procédé selon les revendications 1—2, caractérisé en ce qu'on effectue la première étape en continu.

4. Procédé selon les revendications 1—3, caractérisé en ce qu'on effectue l'ozonolyse à des températures de −80 à +50° C.

5. Procédé selon les revendications 1—4, caractérisé en ce que la concentration de l'isophorone dans le solvant se situe entre 2 et 100%, de préférence cependant entre 3 à 35%.